# EUROPÄISCHE PATENTSCHRIFT

(11) **EP 2 217 579 B1**
(45) Veröffentlichungstag und Bekanntmachung des Hinweises auf die Patenterteilung: **21.09.2011**
(21) Anmeldenummer: 08850383.4
(22) Anmeldetag: 12.11.2008
(51) Int. Cl.: C07D 251/48

(54) **VERFAHREN ZUR HERSTELLUNG VON 3,6-DIHYDRO-1,3,5-TRIAZIN-DERIVATEN**
PROCESS FOR PREPARING 3,6-DIHYDRO-1,3,5-TRIAZINE DERIVATES
MÉTHODE DE PRÉPARATION DE DÉRIVÉS DE 3,6-DIHYDRO-1,3,5-TRIAZINE

(30) Priorität: 13.11.2007 DE 102007054416
(43) Veröffentlichungstag der Anmeldung: 18.08.2010
(73) Patentinhaber: Studiengesellschaft Kohle MbH, 45470 Mülheim an der Ruhr (DE)
(72) Erfinder: LIST, Benjamin, 45470 Mülheim an der Ruhr (DE); CHEN, Xu, West Lafayette, IN 47906 (US)
(86) Internationale Anmeldenummer: PCT/DE2008/001858
(87) Internationale Veröffentlichungsnummer: WO 2009/062483

(56) Entgegenhaltungen:
- EP-A- 1 574 503
- WO-A-01/55122
- WO-A-99/31088
- US-A- 3 287 366

## Beschreibung

Die vorliegende Erfindung betrifft ein Verfahren zur Herstellung von 3,6-Dihydro-1,3,5-triazin-derivaten, worin ein Biguanid mit einem Aldehyd in Gegenwart einer anorganischen und/oder organischen Base umgesetzt wird.

3,6 Dihydro-1,3,5-Triazin-Derivate zeigen pharmakologische Eigenschaften in der Behandlung von pathologischen Erscheinungen, die mit dem Insulinresistenz-Syndrom assoziiert sind. Einige Patente beschreiben die Herstellung von 3,6 Dihydro-1,3,5-triazin-derivaten. Beispielsweise wird in US 3,287,366 die Synthese von Dihydrotriazin mit der folgenden Struktur beschrieben:

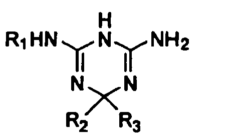

Die Synthese umfasst die Umsetzung eines mono-substituierten Bisguanidins und eines Aldehyds oder Ketons in Gegenwart einer Säure bei erhöhten Temperaturen.

Das japanische Patent JP 480 640 88 beschreibt die Synthese von Dihydrotriazinen mit der folgenden Struktur: 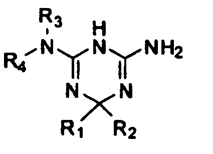 Diese analoge Synthese umfasst das Erhitzen unter sauren Bedingungen.

Das japanische Patent JP54014986 beschreibt die Synthese von Dihydrotriazinen mit der folgenden Struktur: 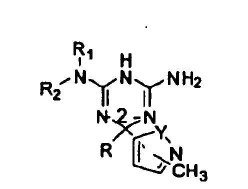 Auch dieses Verfahren erfordert das Erhitzen unter sauren Bedingungen.

In der Patentanmeldung WO01/55122 wird die Synthese von Dihydrotriazinen mit der folgenden Struktur offenbart:

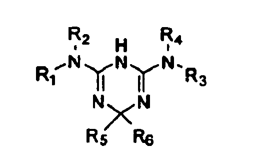

Die Synthese betrifft die Umsetzung von mono-substituierten Bisguanidinen mit einem Acetal, Hemiacetal, Ketal, Hemiketal, Aldehyd oder Keton in Gegenwart einer Säure bei erhöhten Temperaturen.

Den publizierten Verfahren ist gemeinsam, dass eine erhöhte Temperatur notwendig ist, was Rückflussbedingungen oder hoher Druck, wenn Ausgangsverbindungen mit niedrigem Siedepunkt verwendet werden, sowie die Verwendung einer Säure notwendig ist.

Der Erfindung lag die Aufgabe zu Grunde, ein Verfahren zur Herstellung von 3,6-Dihydro-1,3,5-triazin-derivaten zur Verfügung zu stellen, das bei milden Reaktionsbedingungen durchgeführt werden kann. Eine weitere Aufgabe bestand darin, eine Reaktion aufzufinden, in welcher möglichst preiswerte Ausgangsmaterialien zum Einsatz kommen, um die Kosten des Verfahrens zu reduzieren.

Die Erfindung betrifft ein Verfahren zur Herstellung von Verbindungen mit der Formel **I** 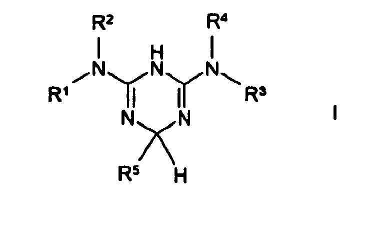 worin R¹, R², R³ und R⁴ unabhängig voneinander ausgewählt sind aus den folgenden Gruppen:
H,
C₁-C₂₀-Alkyl, gegebenenfalls substituiert durch Halogen, C₁-C₅-Alkyl, C₁-C₅-Alkoxy oder C₃-C₈-Cycloalkyl,
C₂-C₂₀-Alkenyl, gegebenenfalls substituiert mit Halogen, C₁-C₅-Alkyl oder C₁-C₅-Alkoxy, C₂-C₂₀-Alkinyl, gegebenenfalls substituiert mit Halogen, C₁-C₅-Alkyl oder C₁-C₅-Alkoxy, C₃-C₈-Cycloalkyl, gegebenenfalls substituiert mit C₁-C₅-Alkyl oder C₁-C₅-Alkoxy, C₃-C₈-Heterocycloalkyl mit einem oder mehreren Heteroatomen ausgewählt aus N, O und S und gegebenenfalls substituiert mit C₁-C₅-Alkyl oder C₁-C₅-Alkoxy,
C₆-C₁₄-Aryl-C₁-C₂₀-alkyl, gegebenenfalls substituiert mit Amino, Hydroxyl, Thio, Halogen, C₁-C₅-Alkyl, C₁-C₅-Alkoxy, C₁-C₅-Alkylthio, C₁-C₅-Alkylamino, C₆-C₁₄-Aryloxy, C₆-C₁₄-Aryl-C₁-C₅-alkoxy, Cyano, Trifluormethyl, Carboxyl, Carboxymethyl oder Carboxyethyl, C₆-C₁₄-Aryl, gegebenenfalls substituiert mit Amino, Hydroxyl, Thio, Halogen, C₁-C₅-Alkyl, C₁-C₅-Alkoxy, C₁-C₁₃-Heteroaryl mit einem oder mehreren Heteroatomen ausgewählt aus N, O und S und gegebenenfalls substituiert mit Amino, Hydroxyl, Thio, Halogen, C₁-C₅-Alkyl, C₁-C₅-Alkoxy, C₁-C₅-Alkylthio, C₁-C₅-Alkylamino, C₆-C₁₄-Aryloxy, C₆-C₁₄-Aryl-C₁-C₅-alkoxy, Cyano, Trifluormethyl, Carboxyl, Carboxymethyl oder Carboxyethyl,
R¹ und R² auf der einen Seite und R³ und R⁴ auf der anderen Seite können mit dem Stickstoffatom am n-gliedrigen Ring (n zwischen 3 und 8) bilden, der gegebenenfalls einen oder mehrere Heteroatome ausgewählt aus N, O und S aufweist und möglicherweise substituiert sein kann durch eine der folgenden Gruppen: Amino, Hydroxyl, Thio, Halogen, C₁-C₅-Alkyl, C₁-C₅-Alkoxy, C₁-C₅-Alkylthio, C₁-C₅-Alkylamino, C₆-C₁₄-Aryloxy, C₆-C₁₄-Aryl-C₁-C₅-alkoxy, Cyano, Trifluormethyl, Carboxyl, Carboxymethyl oder Carboxyethyl;
R⁵ ausgewählt ist aus den folgenden Gruppen:
C₁-C₂₀-Alkyl, gegebenenfalls substituiert mit Amino, Hydroxyl, Thio, Halogen, C₁-C₅-Alkyl, C₁-C₅-Alkoxy, C₁-C₅-Alkylthio, C₁-C₅-Alkylamino, C₆-C₁₄-Aryloxy, C₆-C₁₄-Aryl-C₁-C₅-alkoxy, Cyano, Trifluormethyl, Carboxyl, Carboxymethyl oder Carboxyethyl,
C₁-C₂₀-Alkenyl, gegebenenfalls substituiert mit Amino, Hydroxyl, Thio, Halogen, C₁-C₅-Alkyl, C₁-C₅-Alkoxy, C₁-C₅-Alkylthio, C₁-C₅-Alkylamino, C₆-C₁₄-Aryloxy, C₆-C₁₄-Aryl-C₁-C₅-Alkoxy, Cyano, Trifluormethyl, Carboxyl, Carboxymethyl oder Carboxyethyl,
C₂-C₂₀-Alkinyl, gegebenenfalls substituiert mit Amino, Hydroxyl, Thio, Halogen, C₁-C₅-Alkyl, C₁-C₅-Alkoxy, C₁-C₅-Alkyfthio, C₁-C₅-Alkylamino, C₆-C₁₄-Aryloxy, C₆-C₁₄-Aryl-C₁-C₅-alkoxy, Cyano, Trifluormethyl, Carboxyl, Carboxymethyl oder Carboxyethyl,
C₃-C₈-Cycloalkyl, gegebenenfalls substituiert mit Amino, Hydroxyl, Thio, Halogen, C₁-C₅-Alkyl, C₁-C₅-Alkoxy, C₁-C₅-Alkylthio, C₁-C₅-Alkylamino, C₆-C₁₄-Aryloxy, C₆-C₁₄-Aryl-C₁-C₅-alkoxy, Cyano, Trifluormethyl, Carboxyl, Carboxymethyl oder Carboxyethyl,
C₃-C₈-Heterocycloalkyl mit einem oder mehreren Heteroatomen ausgewählt aus N, O und S und gegebenenfalls substituiert mit Amino, Hydroxyl, Thio, Halogen, C₁-C₅-Alkyl, C₁-C₅-Alkoxy, C₁-C₅-Alkylthio, C₁-C₅-Alkylamino, C₆-C₁₄-Aryloxy, C₆-C₁₄-Aryl-C₁-C₅-Alkoxy, Cyano, Trifluormethyl, Carboxyl, Carboxymethyl oder Carboxyethyl,
C₆-C₁₄-Aryl, gegebenenfalls substituiert mit Amino, Hydroxyl, Thio, Halogen, C₁-C₅-Alkyl, C₁-C₅-Alkoxy, C₁-C₅-Alkylthio, C₁-C₅-Alkylamino, C₆-C₁₄-Aryloxy, C₆-C₁₄-Aryl-C₁-C₅-Alkoxy, Cyano, Trifluormethyl, Carboxyl, Carboxymethyl oder Carboxyethyl,
C₁-C₁₃-Heteroaryl mit einem oder mehreren Heteroatomen ausgewählt aus N, O und S und gegebenenfalls substituiert mit Amino, Hydroxyl, Thio, Halogen, C₁-C₅-Alkyl, C₁-C₅-Alkoxy, C₁-C₅-Alkylthio, C₁-C₅-Alkylamino, C₆-C₁₄-Aryloxy, C₆-C₁₄-Aryl-C₁-C₅-Alkoxy, Cyano, Trifluormethyl, Carboxyl, Carboxymethyl oder Carboxyethyl,
C₆-C₁₄-Aryl-C₁-C₅-alkyl gegebenenfalls substituiert mit Amino, Hydroxyl, Thio, Halogen, C₁ C₅-Alkyl, C₁-C₅-Alkoxy, C₁-C₅-Alkylthio, C₁-C₅-Alkylamino, C₆-C₁₄-Aryloxy, C₆-C₁₄-Aryl-C₁-C₅-Alkoxy, Cyano, Trifluormethyl, Carboxyl, Carboxymethyl oder Carboxyethyl,
   und deren pharmazeutisch geeigneten Derivate, Solvate, Salze und Isomeren,
   **dadurch gekennzeichnet,**
   dass eine Verbindung der Formel II 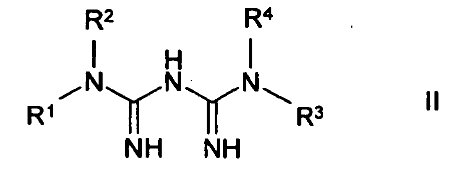 in welcher R¹, R², R³ und R⁴ wie oben definiert sind,
   mit einer Verbindung der Formel III 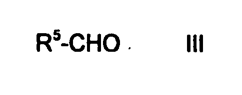 in welcher R⁵ wie oben definiert ist,
   in einem polaren Lösungsmittel oder Lösungsmittelgemisch in Gegenwart einer anorganischen und/oder organischen Base umgesetzt wird.

Überraschenderweise wurde festgestellt, dass das erfindungsgemäße Verfahren eine Reaktionsführung bei Umgebungsdruck und milden Temperaturen ermöglicht. Diese Bedingungen ermöglichen eine ökonomische Verfahrensweise, es wird nicht nur weniger Energie benötigt, auch der apparative Aufwind und die Sicherheit werden verbessert.

Die Base kann ausgewählt sein aus anorganischen Basen, wie Alkalihydroxiden, Alkalicarbonaten und Alkalialkoholaten, z. B. Lithiumhydroxid, Natriumhydroxid, Kaliumhydroxid, Natriumcarbonat, Kaliumcarbonat, Natriummethanolat, Natriumethanolat, etc. oder organischen Basen wie Triethylamin, Diisopropylethylamin, Pyridin, Pyrrol, Pyrrolidin, Piperidin etc. und beliebigen Gemischen der voranstehenden. Besonders gute Ergebnisse hinsichtlich der Ausbeute werden mit starken Basen, insbesondere Alkalihydroxiden etc. erhalten, wobei Natriumhydroxid besonders bevorzugt ist.

Das Verfahren wird üblicherweise in einem Lösungsmittel durchgeführt. Dieses wird vorzugsweise in einer solchen Menge eingesetzt, dass das Reaktionsgemisch gerührt werden kann. Das Lösungsmittel kann aus beliebigen Lösungsmitteln, die die Reaktion nicht negativ beeinflussen ausgewählt werden, wie Wasser, Methanol, Ethanol, Isopropanol, n-Butanol, 2-Butanol, i-Butanol, t-Butanol und N,N-Dimethylformamid etc. oder Mischungen der genannten Lösungsmittel. Als geeignete Lösungsmittel haben sich insbesondere Wasser, Alkohole sowie Gemische aus Wasser und Alkoholen, wie ein Gemisch aus Wasser und Methanol, erwiesen.

Zur Durchführung der Reaktion hat es sich als vorteilhaft erwiesen, wenn die Ausgangsverbindungen mit der Formel II und der Formel III in äquimolaren Mengen bis zu einem Überschuss der Verbindungen mit der III bezogen auf die Verbindung mit der Formel II eingesetzt werden. In einer bevorzugten Ausführungsform beträgt die Konzentration der Verbindung mit der Formel III von 1 Äquivalent bis 10 Äquivalente bezogen auf die Verbindung mit der Formel 11.

Die Menge an zugesetzter Base kann in weiten Bereichen variieren. Vorzugsweise wird die Base in einer Menge von 0,1 Äquivalenten bis 10 Äquivalenten, insbesondere von 0,5 bis 5 Äquivalenten, vorzugsweise von 0,8 bis 2 Äquivalenten, bezogen auf die Verbindung mit der Formel II, eingesetzt. In einer besonders bevorzugten Ausführungsform wird die Base in äquimolaren Mengen bezogen auf die Verbindung mit der Formel II eingesetzt. Der Fachmann wird den Einsatz der Base in Abhängigkeit von den eingesetzten Edukten variieren.

Der Ausdruck "Solvate der Verbindungen" soll Addukte von inerten Lösungsmittelmolekülen an die Verbindungen bedeuten, welche sich aufgrund der wechselseitigen Anziehungskräfte bilden. Solvate sind z.B. Mono- oder Dihydrate oder Alkoholate.

Der Ausdruck "pharmazeutisch verwendbare Derivate" soll z.B. die Salze von Verbindungen gemäß der Erfindung und so genannten Prodrug-Verbindungen bedeuten, wobei "Prodrug-Derivate" üblicherweise Verbindungen mit der Formel I sind, die z.B. mit Alkyl- oder Acylgruppen, Zucker oder Oligopertiden modifiziert wurden und welche im Organismus schnell gespalten werden können, um die aktiven Verbindungen gemäß der Erfindung zu bilden. Das schließt auch bioabbaubare Polymerderivate der Verbindungen gemäß der Erfindung ein, wie sie z.B. in Int. J. Pharm. 115, 61-67 (1995) beschrieben werden.

Zu den Isomeren der Verbindungen mit der Formel I zählen üblicherweise die Stereoisomere und die Tautomeren.

Die erfindungsgemäße Reaktion hat den Vorteil, dass sie bei Umgebungsdruck durchgeführt werden kann. Die Reaktionstemperatur kann von Temperaturen bei -10 °C bis zu erhöhten Temperaturen bis zu 100 °C durchgeführt werden. In einer besonders bevorzugten Ausführungsform wird die Reaktion bei Raumtemperatur durchgeführt. Bei Einsatz von schwächeren Basen kann es von Vorteil sein, die Reaktion bei höheren Temperaturen durchzuführen.

Für alle Reste, die mehr als einmal vorkommen können, wie z.B. Alkyl, optionale Substituenten an Aryl- oder heterocyclischen Resten, sind ihre Bedeutungen und unabhängig voneinander.

Alkyl kann unverzweigt (linear) oder verzweigt sein und hat 1, 2, 3, 4, 5, 6, 7, 8, 9, 10, 11, 12, 13, 14, 15, 16, 17, 18, 19 oder 20 Kohlenstoffatome. Alkyl ist vorzugsweise Methyl, aber auch Ethyl, Propyl, Isopropyl, Butyl, Isobutyl, sec-Botyl oder tert.-Butyl, ebenso Pentyl, 1-, 2-oder 3-Methylpropyl, 1,1-, 1,2- oder 2,2 Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, vorzugsweise aber auch z.B. Trifluormethyl.

Alkyl ist besonders bevorzugt ein Alkyl mit 1, 2, 3, 4, 5 oder 6 Kohlenstoffatomen, vorzugsweise Methyl, Ethyl, Propyl, Isopropyl, Butyl, Isopropyl, sec-Butyl, tert-Butyl, Pentyl, Hexyl, Trifluormethyl, Pentafluroethyl oder 1,1,1-Trifluorethyl.

Cycloalkyl ist vorzugsweise Cyclopropyl, Cyclobutyl, Cyclopentyl, Cyclohexyl oder Cycloheptyl. Alkylen bedeutet vorzugsweise Methylen, Ethylen, Propylen, Butylen, Pentylen oder Hexylen, aber auch verzweigtes Alkylen.

Alkylen ist bevorzugt Vinyl.

Alkinyl ist bevorzugt C≡CH.

Halogen ist F, Cl, G, Br oder I.

Alkoxy ist vorzugsweise Methoxy, Ethoxy, Propoxy oder Butoxy.

C₃-C₈-Heterocycloalkyl mit einem oder mehreren Heteroatomen ausgewählt aus N, O und S ist vorzugsweise 2,3-Dihydro-2-, -3-. -4- oder -5-furyl, 2,5-Dihydro-2-, -3-, -4- oder -5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetra-hydro-1-, -2-, -3-, -4-, -5- oder -6-pyridyl, 1-, 2-, 3- oder 4-piperidinyl, 2-, 3- oder 4-morpholinyl, Tetrahydro-2-, -3- oder -4-pyranyl, 1,4-Dioxanyl, 1,3-Dioxan-2-, -4- oder -5-yl, hexahydro-1-, -3- oder -4-pyridazinyl, Hexahydro-1-, -2-, -4- oder -5-pyrimidinyl, 1-, 2- oder 3-piperazinyl, 1,2,3,4-Tetrahydro-1-, -2-, -3-, -4-, -5-, -6-, -7- oder -8-chinolyl, 1,2,3,4-Tetrahydro-1-, -2-, -3-, -4-, -5-, -6-, -7- oder -8-isochinolyl, 2-, 3-, 5-, 6-, 7- oder 8-3,4-Dihydro-2H-benzo-1,4-oxazinyl.

Gegebenenfalls substituiert bedeutet unsubstituiert oder mono-, di-, tri-, tetra- oder pentasubstituiert.

Aryl ist vorzugsweise Phenyl, Naphthyl oder Diphenyl.

Arylalkyl ist vorzugsweise Benzyl.

Heteroaryl mit einem oder mehreren Heteroatomen ausgewählt aus N, O und S ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4-oder 5-Thiazolyl, 3-, 4- oder 5-Isothiazolyl, 2-, 3- oder 4-Pyridyl, 2-, 4-, 5- oder 6-Pyrimidinyl, außerdem bevorzugt 1,2,3-Triazol-1-, -4- oder -5-yl, 1,2,4-Triazol-1-, -3- oder -5-yl, 1- oder 5-Tetrazolyl, 1,2,3-Oxadiazol-4- oder -5-yl, 1,2,4-Oxadiazol-3- oder -5-yl, 1,3,4-Thiadiazol-2-oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo-1,4-oxazinyl, außerdem bevorzugt 1,3-Benzodioxol-5-yl, 1,4-Benzodioxan-6-yl, 2,1,3-Benzothiadiazol-4-oder -5-yl oder 2,1,3-Benzoxadiazol-5-yl.

R¹, R², R³ und R⁴ sind vorzugsweise unabhängig voneinander aus den folgenden Gruppen ausgewählt:
C₁-C₂₀-Alkyl gegebenenfalls substitutiert mit Halgen, C₁-C₅-Alkyl, C₁-C₅-Alkoxy oder C₃-C₈-Cycloalkyl;
C₂-C₂₀-Alkenyl gegebenfalls substitutiert mit Halogen, C₁-C₅-Alkyl oder C₁-C₅-Alkoxy, C₂-C₂₀-Alkinyl gegebenenfalls substitutiert mit Halgen, C₁-C₅-Alkyl oder C₁-C₅-Alkoxy;
C₃-C₈-Cycloalkyl gegebenenfalls substitutiert mit Halgen, C₁-C₅-Alkyl oder C₁-C₅-Alkoxy;
C₃-C₈-Heterocycloalkyl mit einem oder mehreren Heteroatomen ausgewählt aus N, O and S und gegebenenfalls substitutiert mit C₁-C₅-Alkyl oder C₁-C₅-Alkoxy;
C₆-C₁₄-Aryl-C₁-C₂₀-alkyl gegebenenfalls substituiert mit Amino, Hydroxyl, Thio, Halogen, C₁-C₅-Alkyl, C₁-C₅Alkoxy, C₁C₅-Alkylthio, C₁-C₅-Alkylamino, C₆-C₁₄-Aryloxy, C₆-C₁₄-Aryl, C₁-C₅-Alkoxy, Cyano, Trifluoromethyl, Carboxyl, Carboxymethyl oder Carboxyethyl;
C₆-C₁₄-Aryl gegebenfalls substitutiert mit Amino, Hydroxyl, Thio, Halogen, C₁-C₅-Alkyl, C₁-C₅-Alkoxy,
C₁-C₁₃-Heteroaryl mit einem oder mehreren Heteroatomen ausgewählt aus N, O und S und gegebenenfalls substituiert mit Amino, Hydroxyl, Thio, Halogen, C₁-C₅-Alkyl, C₁-C₅-Alkoxy, C₁-C₅-Alkylthio, C₁-C₅-Alkylamino, C₆-C₁₄-Aryloxy, C₆-C,₄-Aryl, C₁-C₅-Alkoxy, Cyano, Trifluoromethyl, Carboxyl, Carboxymethyl oder Carboxyethyl;
Ein bevorzugtes Verfahren zur Herstellung der Verbindungen mit der Formel I betrifft Verbindungen, in welchen
R¹, R², R³ und R⁴ unabhängig voneinander C₁-C₂₀-Alkyl sind,
R³, R⁴ sind H,
R⁵ ist C₁-C₂₀-Alkyl.

Ein besonders bevorzugtes Verfahren zur Herstellung der Verbindungen mit der Formel I betrifft Verbindungen, in denen
R¹, R², unabhängig voneinander C₁-C₆-Alkyl sind,
R³, R⁴ sind H,
R⁵ ist C₁-C₆-Alkyl.

Ganz besonders bevorzugt ist ein Verfahren zur Herstellung einer Verbindung mit der Formel I
in welcher
R¹, R² Methyl sind,
R³, R⁴ H sind,
R⁵, Methyl ist.

Es wird ein Beispiel zur genaueren Beschreibung der Erfindung gegeben, diese Erfindung ist aber nicht auf das Beispiel beschränkt.

### Beispiel 1:

Herstellung von 2-Amino-3,6-dihydro-4-dimethylamino-6-methyl-1,3,5-triazin:
Ein 4 L Zweihalskolben gefüllt mit 662 g (4 mol) Metformin x HCl (1,21-Dimethylbiguanid x HCl) und 1600 mL MeOH wurde mit einem Magnetrührer mit 250 Upm bei Raumtemperatur in einem Wasserbad gerührt. Zu dieser Suspension wurde über einen Tropftrichter 160 g (4 mol) Natriumhydroxid in 200 mL Wasser hinzugefügt. Zur gleichen Zeit wurden über einen anderen Trichter 226 mL (4 mol) Acetaldehyd in 400 mL MeOH zum Gemisch hinzugefügt.

Die Zugabe der NaOH-Lösung war innerhalb von 70 Minuten abgeschlossen, während die Zugabe von Acetaldehyd innerhalb von 100 Minuten abgeschlossen war. Anschließend wurde das Reaktionsgemisch über Celite gefiltert, um Natriumchlorid zu entfernen. Die Lösung wurde eingeengt, wobei ein weißer Feststoff erhalten wurde, der mit 1,2 L heißem Ethanol extrahiert wurde, wobei eine Suspension erhalten wurde. Nach dem Filtrieren wurde die Ethanollösung unter Bildung eines schwachgelben Feststoffes konzentriert, 520 g, 84 % Ausbeute.
¹H NNR (300 MHz, CDCl₃) 6 1.37 (d, *J* = 6.0 Hz, 3H), 2.98 (s, 6H), 3.46 (s, 1H), 4.83 (q, *J* = 6.0 Hz, 1 H). ¹³C NMR (300 MHz, CDCl₃) δ 24.5, 36.4, 63.0.

### Beispiel 2:

Zu seiner Suspension aus 65 g Metformin x HCl (10 mmol) und 0,4 g (10) mmol Natriumhydroxid in 10 mL Methanol wurde eine Lösung aus 0,56 mL (10 mmol) Acetaldehyd in 4 mL Methanol bei Raumtemperatur über 10 Minuten hinzugefügt, anschließend wurde eine Stunde gerührt. Dann wurde die Lösung abfiltriert und unter Bildung eines weißen Feststoffes konzentriert. Der weiße Feststoff wurde in 10 mL heißem Ethanol gelöst und wieder filtriert. Nach der Konzentration im Vakuum wurden 1,4 g eines weißen Feststoffes erhalten, Ausbeute: 90 %.

### Beispiel 3:

Zu einer Lösung von 1,65 g (10 mmol) Metformin HCL und 0,4 h (10 mmol) Natriumhydroxid in 10 mL Wasser wurde eine Lösung von 0,56 mL (10 mmol) Acetaldehyd in 4 ml Wasser über 10 Minuten hinzugefügt. Nach einer Stunde bei Raumtemperatur wurde die Lösung konzentriert unter Bildung eines weißen Feststoffes. Anschließend wurde der Feststoff in 10 mL Ethanol gelöst und filtriert. Nach Konzentration wurden 1,45 g eines weißen Feststoffes erhalten, Ausbeute 93 %.

## Patentansprüche

1. Verfahren zur Herstellung von Verbindungen mit der Formel I 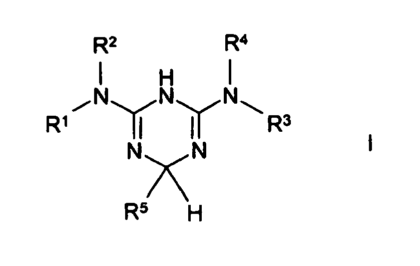 worin R¹, R², R³ und R⁴ unabhängig voneinander ausgewählt sind aus den folgenden Gruppen:
H,
C₁-C₂₀-Alkyl, gegebenenfalls substituiert durch Halogen, C₁-C₅-Alkyl, C₁-C₅-Alkoxy oder
C₃-C₈-Cycloalkyl,
C₂-C₂₀-Alkenyl, gegebenenfalls substituiert mit Halogen, C₁-C₅-Alkyl oder C₁-C₅-Alkoxy, C₂-C₂₀-Alkinyl, gegebenenfalls substituiert mit Halogen, C₁-C₅-Alkyl oder C₁-C₅-Alkoxy, C₃-C₈-Cycloalkyl, gegebenenfalls substituiert mit C₁-C₅-Alkyl oder C₁-C₅-Alkoxy,
C₃-C₈-Heterocycloalkyl mit einem oder mehreren Heteroatomen ausgewählt aus N, O und S und gegebenenfalls substituiert mit C₁-C₅-Alkyl oder C₁-C₅-Alkoxy, C₆-C₁₄-Aryl-C₁-C₂₀-alkyl, gegebenenfalls substituiert mit Amino, Hydroxyl, Thio, Halogen, C₁-C₅-Alkyl, C₁-C₅-Alkoxy, C₁-C₅-Alkylthio, C₁-C₅-Alkylamino, C₆-C₁₄-Aryloxy, C₆-C₁₄-Aryl-C₁-C₅-alkoxy, Cyano, Trifluormethyl, Carboxyl, Carboxymethyl oder Carboxyethyl, C₆-C₁₄-Aryl, gegebenenfalls substituiert mit Amino, Hydroxyl, Thio, Halogen, C₁-C₅-Alkyl, C₁-C₅-Alkoxy,
C₁-C₁₃-Heteroaryl mit einem oder mehreren Heteroatomen ausgewählt aus N, O und S und gegebenenfalls substituiert mit Amino, Hydroxyl, Thio, Halogen, C₁-C₅-Alkyl, C₁-C₅-Alkoxy, C₁-C₅-Alkylthio, C₁-C₅-Alkylamino, C₆-C₁₄-Aryloxy, C₆-C₁₄-Aryl-C₁-C₅-alkoxy, Cyano, Trifluormethyl, Carboxyl, Carboxymethyl oder Carboxyethyl,
R¹ und R² auf der einen Seite und R³ und R⁴ auf der anderen Seite können mit dem Stickstoffatom einen n-gliedrigen Ring (n zwischen 3 und 8) bilden, der gegebenenfalls einen oder mehrere Heteroatome ausgewählt aus N, O und S aufweist und
möglicherweise substituiert sein kann durch eine der folgenden Gruppen: Amino, Hydroxyl, Thio, Halogen, C₁-C₅-Alkyl, C₁-C₅-Alkoxy, C,-C₅-Alkylthio, C₁-C₅-Alkylamino, C₆-C₁₄-Aryloxy, C₆-C₁₄-Aryl-C₁-C₅-alkoxy, Cyano, Trifluormethyl, Carboxyl, Carboxymethyl oder Carboxyethyl;
R⁵ ausgewählt ist aus den folgenden Gruppen:
C₁-C,₂₀-Alkyl, gegebenenfalls substituiert mit Amino, Hydroxyl, Thio, Halogen, C₁-C₅-Alkyl, C₁C₅-Alkoxy, C₁-C₅-Alkylthio, C₁C₅-Alkylamino, C₆-C₁₄-Aryloxy, C₆-C₁₄-Aryl-C₁-C₅-alkoxy, Cyano, Trifluormethyl, Carboxyl, Carboxymethyl oder Carboxyethyl, C₁-C₂₀-Alkenyl, gegebenenfalls substituiert mit Amino, Hydroxyl, Thio, Halogen, C₁-C₅-Alkyl, C₁-C₅-Alkoxy, C₁-C₅-Alkylthio, C₁-C₅-Alkylamino, C₈-C₁₄-Aryloxy, C₆-C₁₄-Aryl-C₁-C₅-Alkoxy, Cyano, Trifluormethyl, Carboxyl, Carboxymethyl oder Carboxyethyl, C₂-C₂₀-Alkinyl, gegebenenfalls substituiert mit Amino, Hydroxyl, Thio, Halogen, C₁-C₅-Alkyl, C₁-C₅-Alkoxy, C₁-C₅-Alkylthio, C₁-C₅-Alkylamino, C₆-C₁₄-Aryloxy, C₆-C₁₄-Aryl-C₁-C₅-alkoxy, Cyano, Trifluormethyl, Carboxyl, Carboxymethyl oder Carboxyethyl, C₃-C₈-Cycloalkyl, gegebenenfalls substituiert mit Amino, Hydroxyl, Thio, Halogen, C₁-C₅-Alkyl, C₁-C₅-Alkoxy, C₁-C₅-Alkylthio, C₁-C₅-Alkylamino, C₆-C₁₄-Aryloxy, C₆-C₁₄-Aryl-Cᵢ-C₅-alkoxy, Cyano, Trifluormethyl, Carboxyl, Carboxymethyl oder Carboxyethyl, C₃-C₈-Heterocycloalkyl mit einem oder mehreren Heteroatomen ausgewählt aus N, O und S und gegebenenfalls substituiert mit Amino, Hydroxyl, Thio, Halogen, C₁-C₅-Alkyl, C₁-C₅-Alkoxy, C₁-C₅-Alkylthio, C₁-C₅-Alkylamino, C₆-C₁₄-Aryloxy, C₆-C₁₄-Aryl-C₁-C₅-Alkoxy, Cyano, Trifluormethyl, Carboxyl, Carboxymethyl oder Carboxyethyl, C₆-C₁₄-Aryl, gegebenenfalls substituiert mit Amino, Hydroxyl, Thio, Halogen, C₁-C₅-Alkyl, C₁-C₅-Alkoxy, C₁-C₅,-Alkylthio, C₁-C₅-Alkylamino, C₆-C₁₄-Aryloxy, C₆-C₁₄-Aryl-C₁-C₅-Alkoxy, Cyano, Trifluormethyl, Carboxyl, Carboxymethyl oder Carboxyethyl, C₁-C₁₃-Heteroaryl mit einem oder mehreren Heteroatomen ausgewählt aus N, O und S und gegebenenfalls substituiert mit Amino, Hydroxyl, Thio, Halogen, C₁-C₅-Alkyl, C₁-C₅-Alkoxy, C₁-C₅-Alkylthio, C₁-C₅-Alkylamino, C₆-C₁₄-Aryloxy, C₆-C₁₄-Aryl-C₁-C₅-Alkoxy, Cyano, Trifluormethyl, Carboxyl, Carboxymethyl oder Carboxyethyl, C₆-C₁₄-Aryl-C₁-C₅-alkyl gegebenenfalls substituiert mit Amino, Hydroxyl, Thio, Halogen, C₁-C₅-Alkyl, C₁-C₅-Alkoxy, C₁-C₅-Alkylthio, C₁-C₅-Alkylamino, C₁-C₁₄-Aryloxy, C₆-C₁₄-Aryl-C₁-C₅-Alkoxy, Cyano, Trifluormethyl, Carboxyl, Carboxymethyl oder Carboxyethyl, und deren pharmazeutisch geeigneten Derivate, Solvate, Salze und Isomeren, **dadurch gekennzeichnet,**
**dass** eine Verbindung der Formel II 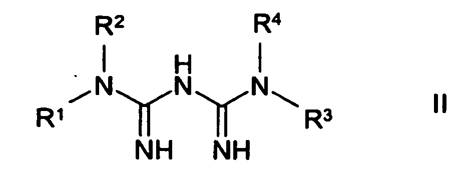 in welcher R¹, R², R³ und R⁴ wie oben definiert sind,
mit einer Verbindung der Formel III 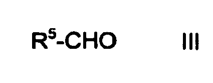 in welcher R⁵ wie oben definiert ist,
in einem polaren Lösungsmittel oder Lösungsmittelgemisch in Gegenwart einer anorganischen und/oder organischen Base umgesetzt wird.

2. Verfahren nach Anspruch 1,
in welchem R¹, R² unabhängig voneinander C₁-C₂₀-Alkyl sind, R³, R⁴ H sind,
R⁵ C₁-C₂₀-Alkyl ist,

3. Verfahren nach Anspruch 1 oder 2,
in welchem R¹, R² unabhängig voneinander C₁-C₆-Alkyl sind, R³, R⁴ H sind,
R⁵ C₁-C₆-Alkyl ist.

4. Verfahren nach Anspruch 1, 2 oder 3,
in welchem R¹, R² Methyl sind,
R³, R⁴ H sind,
R⁵ Methyl ist.

5. Verfahren nach einem oder mehreren der Ansprüche 1 bis 4,
in welchem die Base ausgewählt ist aus der Gruppe aus Alkalihydroxiden, Alkalicarbonaten, Alkalialkoholaten, oder organischen Basen wie Triethylamin, Diisopropylethylamin, Pyridin, Pyrrol, Pyrrolidin, Piperidin und beliebigen Gemischen der voranstehenden.

6. Verfahren nach einem oder mehreren der Ansprüche der 1 bis 5,
in welchem das Lösungsmittel ausgewählt ist aus der Gruppe bestehend aus Wasser, Methanol, Ethanol, Isopropanol, n-Butanol, 2-Butanol, i-Butanol, t-Butanol, NN-Dimethylformamid oder Mischungen der genannten Lösungsmittel.

7. Verfahren nach einem oder mehreren der Ansprüche 1 bis 6,
in welchem die Verbindung mit der Formel III von 1 Äquivalent bis 10 Äquivalenten, bezogen auf die Verbindung mit der Formel II, eingesetzt wird.

8. Verfahren nach einem oder mehreren der Ansprüche 1 bis 7,
worin die Base von 0,1 Äquivalenten bis 10 Äquivalenten bezogen auf die Verbindung mit der Formel II vorliegt.

9. Verfahren nach einem oder mehreren der Ansprüche 1 bis 8,
in welchem die Verbindung mit der Formel III Acetaldeyhd ist.

10. Verfahren nach einem oder mehreren der Ansprüche 1 bis 9,
in welchem die Reaktion unter Umgebungsdruck durchgeführt wird.

11. Verfahren nach einem oder mehreren der Ansprüche 1 bis 10,
in welchem die Reaktion bei Raumtemperatur durchgeführt wird.

## Claims

1. Process for preparing compounds with the formula I 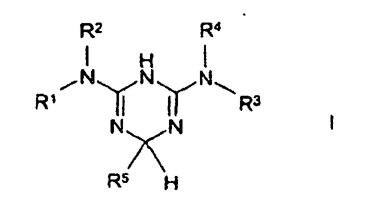 in which R¹, R², R³ and R⁴ are each independently selected from the following groups:
H,
C₁-C₂₀-alkyl optionally substituted by halogen, C₁-C₅-alkyl, C₁-C₅-alkoxy or C₃-C₈-cycloalkyl,
C₂-C₂₀-alkenyl optionally substituted by halogen,
C₁-C₅-alkyl or C₁-C₅-alkoxy,
C₂-C₂₀-alkynyl optionally substituted by halogen,
C₁-C₅-alkyl or C₁-C₅-alkoxy,
C₃-C₈-cycloalkyl optionally substituted by C₁-C₅-alkyl or C₁-C₅-alkoxy,
C₃-C₈-heterocycloalkyl with one or more heteroatoms selected from N, O and S, and optionally substituted by C₁-C₅-alkyl or C₁-C₅-alkoxy,
C₆-C₁₄-aryl-C₁-C₂₀-alkyl optionally substituted by amino, hydroxyl, thio, halogen, C₁-C₅-alkyl, C₁-C₅-alkoxy, C₁-C₅-alkylthio, C₁-C₅-alkylamino, C₆-C₁₄-aryloxy, C₆-C₁₄-aryl-C₁-C₅-alkoxy, cyano, trifluoromethyl, carboxyl, carboxymethyl or carboxyethyl, C₆-C₁₄-aryl optionally substituted by amino, hydroxyl, thio, halogen, C₁-C₅-alkyl, C₁-C₅-alkoxy, C₁-C₁₃-heteroaryl with one or more heteroatoms selected from N, O and S and optionally substituted by amino, hydroxyl, thio, halogen, C₁-C₅-alkyl, C₁-C₅-alkoxy, C₁-C₅-alkylthio, C₁-C₅-alkylamino, C₆-C₁₄-aryloxy, C₆-C₁₄-aryl-C₁-C₅-alkoxy, cyano, trifluoromethyl, carboxyl, carboxymethyl or carboxyethyl,
R¹ and R² on the one hand, and R³ and R⁴ on the other hand, may form, with the nitrogen atom, an n-membered ring (n from 3 to 8) which optionally has one or more heteroatoms selected from N, O and
S and may possibly be substituted by one of the following groups: amino, hydroxyl, thio, halogen, C₁-C₅-alkyl, C₁-C₅-alkoxy, C₁-C₅-alkylthio, C₁-C₅-alkylamino, C₆-C₁₄-aryloxy, C₆-C₁₄-aryl-C₁-C₅-alkoxy, cyano, trifluoromethyl, carboxyl, carboxymethyl or carboxyethyl;
R⁵ is selected from the following groups:
C₁-C₂₀-alkyl optionally substituted by amino, hydroxyl, thio, halogen, C₁-C₅-alkyl , C₁-C₅-alkoxy, C₁-C₅-alkylthio, C₁-C₅-alkylamino, C₆-C₁₄-aryloxy, C₆-C₁₄-aryl-C₁-C₅-alkoxy, cyano, trifluoromethyl, carboxyl, carboxymethyl or carboxyethyl,
C₁-C₂₀-alkenyl optionally substituted by amino, hydroxyl, thio, halogen, C₁-C₅-alkyl, C₁-C₅-alkoxy, C₁-C₅-alkylthio, C₁-C₅-alkylamino, C₆-C₁₄-aryloxy, C₆-C₁₄-aryl-C₁-C₅-alkoxy, cyano, trifluoromethyl, carboxyl, carboxymethyl or carboxyethyl,
C₂-C₂₀-alkynyl optionally substituted by amino, hydroxyl, thio, halogen, C₁-C₅-alkyl, C₁-C₅-alkoxy, C₁-C₅-alkylthio, C₁-C₅-alkylamino, C₆-C₁₄-aryloxy, C₆-C₁₄-aryl-C₁-C₅-alkoxy, cyano, trifluoromethyl, carboxyl, carboxymethyl or carboxyethyl,
C₃-C₈-cycloalkyl optionally substituted by amino, hydroxyl, thio, halogen, C₁-C₅-alkyl, C₁-C₅-alkoxy, C₁-C₅-alkylthio, C₁-C₅-alkylamino, C₆-C₁₄-aryloxy, C₆-C₁₄-aryl-C₁-C₅-alkoxy, cyano, trifluoromethyl, carboxyl, carboxymethyl or carboxyethyl,
C₃-C₈-heterocycloalkyl with one or more heteroatoms selected from N, O and S, and optionally substituted by amino, hydroxyl, thio, halogen, C₁-C₅-alkyl, C₁-C₅-alkoxy, C₁-C₅-alkylthio, C₁-C₅-alkylamino, C₆-C₁₄-aryloxy, C₆-C₁₄-aryl-C₁-C₅-alkoxy, cyano, trifluoromethyl, carboxyl, carboxymethyl or carboxyethyl,
C₆-C₁₄-aryl optionally substituted by amino, hydroxyl, thio, halogen, C₁-C₅-alkyl, C₁-C₅-alkoxy, C₁-C₅-alkylthio, C₁-C₅-alkylamino, C₆-C₁₄-aryloxy, C₆-C₁₄-aryl-C₁-C₅-alkoxy, cyano, trifluoromethyl, carboxyl, carboxymethyl or carboxyethyl,
C₁-C₁₃-heteroaryl with one or more heteroatoms selected from N, O and S, and optionally substituted by amino, hydroxyl, thio, halogen, C₁-C₅-alkyl, C₁-C₅-alkoxy, C₁-C₅-alkylthio, C₁-C₅-alkylamino, C₆-C₁₄-aryloxy, C₆-C₁₄-aryl-C₁-C₅-alkoxy, cyano, trifluoromethyl, carboxyl, carboxymethyl or carboxyethyl,
C₆-C₁₄-aryl-C₁-C₅-alkyl optionally substituted by amino, hydroxyl, thio, halogen, C₁-C₅-alkyl, C₁-C₅-alkoxy, C₁-C₅-alkylthio, C₁-C₅-alkylamino, C₆-C₁₄-aryloxy, C₆-C₁₄-aryl-C₁-C₅-alkoxy, cyano, trifluoromethyl, carboxyl, carboxymethyl or carboxyethyl,
and the pharmaceutically acceptable derivatives, solvates, salts and isomers thereof,
**characterized in that**
a compound of the formula II 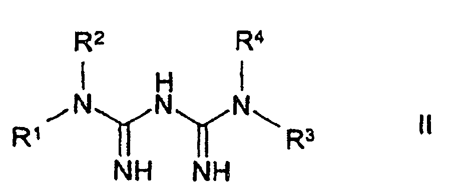 in which R¹, R², R³ and R⁴ are each as defined above is reacted with a compound of the formula III 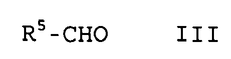 in which R⁵ is as defined above
in a polar solvent or solvent mixture in the presence of an inorganic and/or organic base.

2. Process according to Claim 1,
in which R¹, R² are each independently C₁-C₂₀-alkyl, R³, R⁴ are each H,
R⁵ is C₁-C₂₀-alkyl.

3. Process according to Claim 1 or 2,
in which R¹, R² are each independently C₁-C₆-alkyl, R³, R⁴ are each H,
R⁵ is C₁-C₆-alkyl.

4. Process according to Claim 1, 2 or 3,
in which R¹, R² are each methyl,
R³, R⁴ are each H,
R⁵ is methyl.

5. Process according to one or more of Claims 1 to 4, in which the base is selected from the group of alkali metal hydroxides, alkali metal carbonates, alkali metal alkoxides, or organic bases such as triethylamine, diisopropylethylamine, pyridine, pyrrole, pyrrolidine, piperidine and any desired mixtures of the above.

6. Process according to one or more of Claims 1 to 5, in which the solvent is selected from the group consisting of water, methanol, ethanol, isopropanol, n-butanol, 2-butanol, i-butanol, t-butanol, N,N-dimethylformamide or mixtures of the solvents mentioned.

7. Process according to one or more of Claims 1 to 6, in which from 1 equivalent to 10 equivalents of the compound with the formula III are used, based on the compound with the formula II.

8. Process according to one or more of Claims 1 to 7, in which from 0.1 equivalent to 10 equivalents of the base are present, based on the compound with the formula II.

9. Process according to one or more of Claims 1 to 8, in which the compound of the formula III is acetaldehyde.

10. Process according to one or more of Claims 1 to 9, in which the reaction is conducted under ambient pressure.

11. Process according to one or more of Claims 1 to 10,
in which the reaction is conducted at room temperature.

## Revendications

1. Procédé pour la préparation de composés de formule 1, 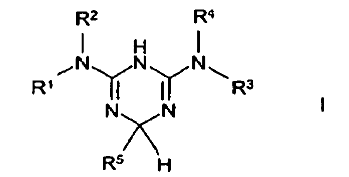 où R¹, R² R³ et R⁴ sont choisis, indépendamment l'un de l'autre, dans les groupes suivants :
H,
C₁-C₂₀-alkyle, le cas échéant substitué par halogène, C₁-C₅-alkyle, C₁-C₅-alcoxy ou C₃-C₈-cycloalkyle, C₂-C₂₀-alcényle, le cas échéant substitué par halogène,
C₁-C₅-alkyle ou C₁-C₅-alcoxy,
C₂-C₂₀-alcynyle, le cas échéant substitué par halogène,
C₁-C₅-alkyle ou C₁-C₅-alcoxy,
C₃-C₅-cycloalkyle, le cas échéant substitué par C₁-C₅-alkyle ou C₁-C₅-alcoxy,
C₃-C₈-hétérocycloalkyle comprenant un ou plusieurs hétéroatomes choisis parmi N, O et S et le cas échéant substitué par C₁-C₅-alkyle ou C₁-C₅-alcoxy, C₆-C₁₄-aryl-C₁-C₂₀-alkyle, le cas échéant substitué par amino, hydroxyle, thio, halogène, C₁-C₅-alkyle, C₁-C₅-alcoxy, C₁-C₅-alkylthio, C₁-C₅-alkylamino, C₆-C₁₄-aryloxy, C₆-C₁₄-aryl-C₁-C₅-alcoxy, cyano, trifluorométhyle, carboxyle, carboxyméthyle ou carboxyéthyle, C₆-C₁₄-aryle, le cas échéant substitué par amino, hydroxyle, thio, halogène, C₁-C₅-alkyle, C₁-C₅-alcoxy, C₁-C₁₃-hétéroaryle comprenant un ou plusieurs hétéroatomes choisis parmi N, O et S et le cas échéant substitué par amino, hydroxyle, thio, halogène, C₁-C₅-alkyle, C₁-C₅-alcoxy, C₁-C₅-alkylthio, C₁-C₅-alkylamino, C₆-C₁₄-aryloxy, C₆-C₁₄-aryl-C₁-C₅-alcoxy, cyano, trifluorométhyle, carboxyle, carboxyméthyle ou carboxyéthyle,
R¹ et R² d'une part et R³ et R⁴ d'autre part peuvent former avec l'atome d'azote un cycle à n chaînons (n compris entre 3 et 8), qui présente le cas échéant un ou plusieurs hétéroatomes choisis parmi N, O et S et qui peut éventuellement être substitué par un des groupes suivants : amino, hydroxyle, thio, halogène, C₁-C₅-alkyle, C₁-C₅-alcoxy, C₁-C₅-alkylthio, C₁-C₅-alkylamino, C₆-C₁₄-aryloxy, C₆-C₁₄-aryl-C₁-C₅-alcoxy, cyano, trifluorométhyle, carboxyle, carboxyméthyle ou carboxyéthyle ;
R⁵ est choisi dans les groupes suivants :
C₁-C₂₀-alkyle, le cas échéant substitué par amino, hydroxyle, thio, halogène, C₁-C₅-alkyle, C₁-C₅-alcoxy, C₁-C₅-alkylthio, C₁-C₅-alkylamino, C₆-C₁₄-aryloxy, C₆-C₁₄-aryl-C₁-C₅-alcoxy, cyano, trifluorométhyle, carboxyle, carboxyméthyle ou carboxyéthyle,
C₁-C₂₀-alcényle, le cas échéant substitué par amino, hydroxyle, thio, halogène, C₁-C₅-alkyle, C₁-C₅-alcoxy, C₁-C₅-alkylthio, C₁-C₅-alkylamino, C₆-C₁₄-aryloxy, C₆-C₁₄-aryl-C₁-C₅-alcoxy, cyano, trifluorométhyle, carboxyle, carboxyméthyle ou carboxyéthyle,
C₂-C₂₀-alcynyle, le cas échéant substitué par amino, hydroxyle, thio, halogène, C₁-C₅-alkyle, C₁-C₅-alcoxy, C₁-C₅-alkylthio, C₁-C₅-alkylamino, C₆-C₁₄-aryloxy, C₆-C₁₄-aryl-C₁-C₅-alcoxy, cyano, trifluorométhyle, carboxyle, carboxyméthyle ou carboxyéthyle,
C₃-C₈-cycloalkyle, le cas échéant substitué par amino, hydroxyle, thio, halogène, C₁-C₅-alkyle, C₁-C₅-alcoxy, C₁-C₅-alkylthio, C₁-C₅-alkylamino, C₆-C₁₄-aryloxy, C₆-C₁₄-aryl-C₁-C₅-alcoxy, cyano, trifluorométhyle, carboxyle, carboxyméthyle ou carboxyéthyle,
C₃-C₈-hétérocycloalkyle comprenant un ou plusieurs hétéroatomes choisis parmi N, O et S et le cas échéant substitué par amino, hydroxyle, thio, halogène, C₁-C₅-alkyle, C₁-C₅-alcoxy, C₁-C₅-alkylthio, C₁-C₅-alkylamino, C₆-C₁₄-aryloxy, C₆-C₁₄-aryl-C₁-C₅-alcoxy, cyano, trifluorométhyle, carboxyle, carboxyméthyle ou carboxyéthyle,
C₆-C₁₄-aryle, le cas échéant substitué par amino, hydroxyle, thio, halogène, C₁-C₅-alkyle, C₁-C₅-alcoxy, C₁-C₅-alkylthio, C₁-C₅-alkylamino, C₆-C₁₄-aryloxy, C₆-C₁₄-aryl-C₁-C₅-alcoxy, cyano, trifluorométhyle, carboxyle, carboxyméthyle ou carboxyéthyle,
C₁-C₁₃-hétéroaryle comprenant un ou plusieurs hétéroatomes choisis parmi N, O et S et le cas échéant substitué par amino, hydroxyle, thio, halogène, C₁-C₅-alkyle, C₁-C₅-alcoxy, C₁-C₅-alkylthio, C₁-C₅-alkylamino, C₆-C₁₄-aryloxy, C₆-C₁₄-aryl-C₁-C₅-alcoxy, cyano, trifluorométhyle, carboxyle, carboxyméthyle ou carboxyéthyle,
C₆-C₁₄-aryl-C₁-C₅-alkyle, le cas échéant substitué par amino, hydroxyle, thio, halogène, C₁-C₅-alkyle, C₁-C₅-alcoxy, C₁-C₅-alkylthio, C₁-C₅-alkylamino, C₆-C₁₄-aryloxy, C₆-C₁₄-aryl-C₁-C₅-alcoxy, cyano, trifluorométhyle, carboxyle, carboxyméthyle ou carboxyéthyle,
et leurs dérivés, solvates, sels et isomères pharmaceutiquement appropriés, **caractérisé**
**en ce qu'**on transforme un composé de formule II 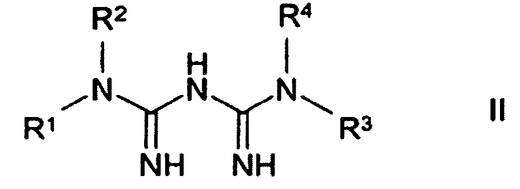 où R¹, R², R³ et R⁴ sont définis comme ci-dessus,
avec un composé de formule III
R⁵-CHO III
où R⁵ est défini comme ci-dessus,
dans un solvant polaire ou un mélange de solvants polaires en présence d'une base inorganique et/ou organique.

2. Procédé selon la revendication 1, dans lequel R¹, R² représentent, indépendamment l'un de l'autre, C₁-C₂₀-alkyle,
R³, R⁴ représentent H,
R⁵ représente C₁-C₂₀-alkyle.

3. Procédé selon la revendication 1 ou 2,
dans lequel R¹, R² représentent, indépendamment l'un de l'autre, C₁-C₆-alkyle,
R³, R⁴ représentent H,
R⁵ représente C₁-C₆-alkyle.

4. Procédé selon la revendication 1, 2 ou 3,
dans lequel R¹, R² représentent méthyle,
R³, R⁴ représentent H,
R⁵ représente méthyle.

5. Procédé selon l'une ou plusieurs des revendications 1 à 4, dans lequel la base est choisie dans le groupe formé par les hydroxydes de métal alcalin, les carbonates de métal alcalin, les alcoolates de métal alcalin ou les bases organiques telles que la triéthylamine, la diisopropyléthylamine, la pyridine, le pyrrole, la pyrrolidine, la pipéridine et les mélanges quelconques des composés susmentionnés.

6. Procédé selon l'une ou plusieurs des revendications 1 à 5, dans lequel le solvant est choisi dans le groupe constitué par l'eau, le méthanol, l'éthanol, l'isopropanol, le n-butanol, le 2-butanol, l'i-butanol, le t-butanol, le N,N-diméthylformamide ou les mélanges des solvants mentionnés.

7. Procédé selon l'une ou plusieurs des revendications 1 à 6, dans lequel le composé de formule III est utilisé à raison de 1 équivalent à 10 équivalents par rapport au composé de formule II.

8. Procédé selon l'une ou plusieurs des revendications 1 à 7, dans lequel la base se trouve à raison de 0,1 équivalent à 10 équivalents par rapport au composé de formule II.

9. Procédé selon l'une ou plusieurs des revendications 1 à 8, dans lequel le composé de formule III est l'acétaldéhyde.

10. Procédé selon l'une ou plusieurs des revendications 1 à 9, dans lequel la réaction est réalisée à pression environnante.

11. Procédé selon l'une ou plusieurs des revendications 1 à 10, dans lequel la réaction est réalisée à température ambiante.
